# EUROPEAN PATENT APPLICATION

(11) **EP 1 674 090 A1**
(43) Date of publication of application: **28.06.2006**
(21) Application number: 04788031.5
(22) Date of filing: 22.09.2004
(51) Int. Cl.: A61K 31/19, A61K 31/215, A61K 31/704, A61P 3/04, A61P 3/06, A61P 5/50, A61P 9/12, A23L 1/30, A23K 1/16, A24B 15/10

(54) **EARLY INSULIN SECRETION PROMOTER**

(30) Priority: 22.09.2003 US 503893 P; 22.09.2003 US 503892 P; 26.12.2003 JP 2003434627; 27.02.2004 JP 2004055440; 27.02.2004 JP 2004055439; 01.03.2004 JP 2004056876; 21.04.2004 JP 2004126091
(71) Applicant: Use-Techno Corporation, Fukuchiyama-shi, Kyoto 6200055 (JP)
(72) Inventor: MATSUYAMA, Futoshi, 6208502 (JP); SEINO, Yutaka, 6610003 (JP); FUKUSHIMA, Mitsuo, Kobe-shi Hyogo 657-0061 (JP); MIURA, Toshihiro, 5100206 (JP); FUJITA, Takeshi, 6650807 (JP); KANEKO, Tetsuo, Kure-shi, Hiroshima 7370112 (JP)
(74) Representative: Ouzman, Beverley Nicola Claire
(86) International application number: PCT/JP2004/013848
(87) International publication number: WO 2005/027892

(57) **Abstract**

The invention provides an early insulin secretion stimulator consisting of corosolic acid, etc. The early insulin secretion stimulator of the invention is capable of rapidly inducing secretion of insulin immediately after meals without inducing secretion of excess insulin in the absence of blood glucose increase.

## Description

### Technical Field

The present invention relates to an early insulin secretion stimulator and to a process for its production, as well as to a pharmaceutical, a health supplement, a smoking material, a food material and an animal feed using the early insulin secretion stimulator.

### Background Art

Banaba (*Lagerstroemia speciosa Linn*. or *Pers.)* is a plant of the family *Lythrum* which is found widely in South East Asian countries including the Philippines, India, Malaysia, Southern China and Australia. Patent Document 1 proposes an antidiabetic agent composed mainly of banaba extract obtained from banaba leaves using hot water or an organic solvent, and the antidiabetic effect has been confirmed in animal experiments with diabetic mice.
Patent Document 1: Japanese Patent Application Laid-Open No. 5-310587.

### Disclosure of the Invention

### Problem to be Solved by the Invention

For treatment of diabetes it is ideal to achieve rapid secretion of insulin immediately after meals while avoiding oversecretion of insulin in the absence of blood glucose increase. However, the current antidiabetic agents, or synthetic drugs for diabetes treatment such as sulfonylurea agents, biguanide agents, thiazolidine derivatives and phenylalanine derivatives have not been able to readily achieve such ideal blood glucose increase suppression and insulin secretion control.

These antidiabetic agents and synthetic drugs, while successfully lowering blood glucose levels, tend to cause hypoglycemia or can provoke reduced insulin sensitivity and insulin resistance, which reduce the effect of insulin, and in some cases may have side effects on the liver, while exhaustion of the pancreas, an insulin secreting organ, has been a particular unavoidable problem.

It is an object of the present invention to provide an early insulin secretion stimulator with low side effects, which rapidly stimulates early insulin secretion and suppresses blood glucose increase only at mealtimes, and is therefore able to exhibit ideal blood glucose increase suppression and insulin secretion control, and a process for its production. It is another object of the invention to provide a pharmaceutical, a health supplement, a smoking material, a food material and an animal feed which employ the early insulin secretion stimulator.

### Means for Solving the Problem

The present inventors have extracted corosolic acid and analogues thereof from banaba, a typical plant containing corosolic acid, and in the course of examining their pharmacological action, have confirmed that corosolic acid and specific analogues thereof have hypoglycemic effects. Moreover it was found, surprisingly, that these compounds also have an effect of stimulating insulin secretion immediately after blood glucose level increase (early insulin secretion stimulating effect).

In other words, the early insulin secretion stimulator of the invention comprises one or more medicinal components selected from the group consisting of corosolic acid, acyloxycorosolic acids, maslinic acid and acyloxymaslinic acids at a content of at least 99 wt% of the total weight. The early insulin secretion stimulator stimulates early secretion of insulin, and the secreted insulin rapidly lowers blood glucose. The rapid fall in blood glucose in turn results in prompt reduction of insulin secretion, thereby finally suppressing oversecretion of insulin.

The early insulin secretion stimulator of the invention is characterized by rapidly stimulating early secretion of insulin only at mealtimes (and especially only with glucose intake), thereby suppressing blood glucose increase. It is therefore possible to achieve ideal blood glucose increase suppression and insulin secretion control, while minimizing side effects such as hypoglycemia.

The one or more medicinal components selected from the group consisting of corosolic acid, acyloxycorosolic acids, maslinic acid and acyloxymaslinic acids are preferably corosolic acid or maslinic acid (more preferably corosolic acid), and the early insulin secretion stimulator of the invention preferably comprises such medicinal components at 100 wt% of the total weight. That is to say, the early insulin secretion stimulator of the invention is preferably composed entirely of corosolic acid or maslinic acid (preferably corosolic acid). The early insulin secretion stimulator of the invention preferably comprises corosolic acid at 99 wt% of the total weight and maslinic acid at less than 1 wt% of the total weight.

Acyloxycorosolic acids such as acetylcorosolic acid are derivatives of corosolic acid and acyloxymaslinic acids such as acetylmaslinic acid are derivatives of maslinic acid, and these also function as early insulin secretion stimulators exhibiting the same function and effect as corosolic acid and maslinic acid.

Hypoglycemic effects are commonly exhibited by various mechanisms, and are not necessarily related to insulin secretion. For example, biguanide agents do not stimulate insulin secretion but rather exhibit a hypoglycemic effect by inhibiting gluconeogenesis in the liver. Also, α-glucosidase inhibitors exhibit a hypoglycemic effect by blocking absorption of glucose through the digestive organs. Thus, compounds with hypoglycemic effects are not necessarily compounds connected with insulin secretion.

Corosolic acid is extracted from loquat (*Eriobotrya japonica*) leaves and has been reported to exhibit a hypoglycemic effect (Planta Medica, 57, 414-416(1991)), but when the present inventors compared the extract from loquat described in this publication with corosolic acid (extracted from banaba), it was found that both have different retention times in high performance liquid chromatography (HPLC). That is, the extract from loquat is a different compound from corosolic acid. Also, this publication does not indicate a connection between the extract and insulin secretion. Thus, absolutely no knowledge has existed in the prior art regarding corosolic acid in connection with early insulin secretion.

Sulfonylurea agents and nateglinide are also know as drug gents associated with insulin secretion. These drugs work not only during times of glucose intake but also often lead to hypoglycemia and are indicated as having the risk of serious side-effects. Exhaustion of the pancreas is also an unavoidable result.

In contrast, the early insulin secretion stimulator of the invention described above is a glucose-dependent early insulin secretion stimulator which stimulates early insulin secretion only during times of glucose intake, and therefore it has few side-effects and minimal burden on the pancreas.

The present inventors have confirmed that the aforementioned effect is produced when the early insulin secretion stimulator of the invention is (1) a glucoside, (2) an ester or (3) a mixture of 100 parts by weight of an early insulin secretion stimulator and 1-99 parts by weight of ursolic acid and oleanolic acid.

Thus, pharmaceuticals (including tablets, capsules, powders, liquids, and gas agents), as well as smoking materials or health supplements, which contain the aforementioned compounds and compositions (early insulin secretion stimulators) as active components can be provided. The aforementioned compounds and compositions may also be added to materials selected from the group consisting of bread, noodles, confectioneries, beverages, sugar, alcoholic beverages, fats and oils, wheat flour, starches, and the like, to prepare food materials.

The early insulin secretion stimulator according to the invention has few side-effects, does not exhaust the pancreas and inhibits blood glucose level increase, and can therefore contribute to prophylactic medicine not only for diabetic patients but also for borderline diabetic patients who are at risk for diabetes.

Rapid secretion of insulin immediately after meals to suppress blood glucose increase, thus preventing oversecretion of insulin, is associated not only with a hypoglycemic effect but also with preventing insulin resistance, preventing obesity due to insulin oversecretion, suppressing triglycerides and preventing the consequent hypertension and arteriosclerosis caused by accumulation of cholesterol.

Administering the early insulin secretion stimulator before, with, after or between meals, or by ingesting food containing the early insulin secretion stimulator, is highly effective for both the treatment and prevention of diabetes, hypertension, hyperlipidemia and obesity and can therefore contribute to maintenance of health.

Corosolic acid, which is used for the early insulin secretion stimulator of the invention, may be obtained by a production process comprising a step of deacylating an acyloxycorosolic acid represented by the following general formula (1). 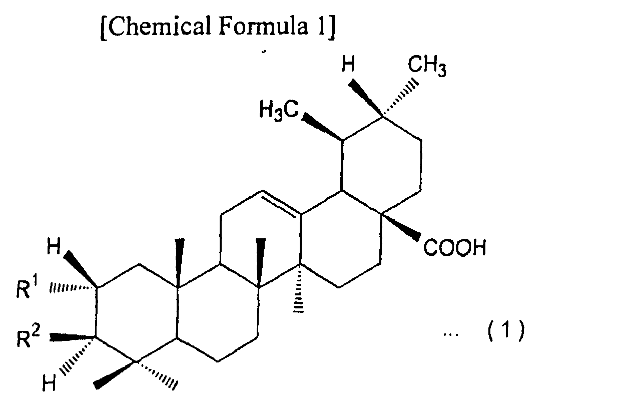 In formula (1), R¹ and R² each independently represent an acyloxy group or hydroxyl group, with the proviso that R¹ and R² are not both hydroxyl groups.

Deacylation of an acyloxycorosolic acid shown above can yield corosolic acid at a very high purity (approximately 100%).

Maslinic acid, which is used for the early insulin secretion stimulator of the invention, may be obtained by a production process comprising a step of deacylating an acyloxymaslinic acid represented by the following general formula (2). 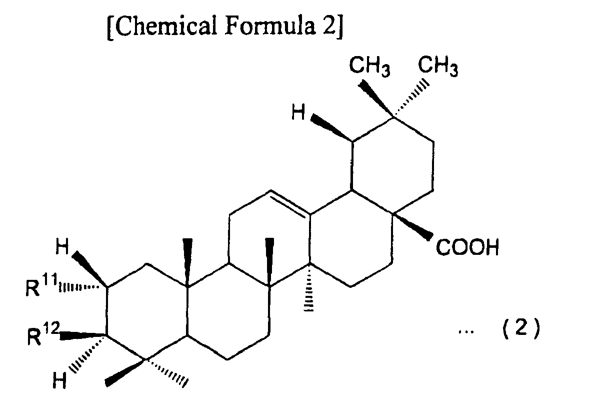 In formula (2), R¹¹ and R¹² each independently represent an acyloxy group or hydroxyl group, with the proviso that R¹¹ and R¹² are not both hydroxyl groups.

Deacylation of an acyloxymaslinic acid shown above can yield maslinic acid at a very high purity (approximately 100%).

### Effects of the Invention

There is provided an early insulin secretion stimulator having the effect of inhibiting blood glucose increase, improving insulin resistance, preventing obesity and suppressing triglycerides by inducing rapid secretion of insulin immediately after meals without inducing secretion of excess insulin in the absence of blood glucose increase. That is, there is provided an early insulin secretion stimulator which stimulates early secretion of insulin to lower postprandial blood glucose levels while preventing excess insulin secretion.

The early insulin secretion stimulator may be administered before, with or after meals, in order to prevent diseases caused by high blood glucose and maintain health, by inhibiting blood glucose level increase, preventing obesity, suppressing triglycerides and preventing insulin resistance.

The early insulin secretion stimulator may be used in the forms of tablets, capsules, injections, drink, gas, patches, suppositories, bath salts and the like, and may be added as a component in bread, noodles, beverages, alcoholic drinks, feeds and smoking materials, for use in ordinary foods, consumer goods and other ordinary products.

### Brief Description of the Drawings

Fig. 1 is a graph showing blood insulin levels during a glucose tolerance test, wherein (a) is a case with administration of corosolic acid and (b) is a case with administration of a placebo.
Fig. 2 is a graph showing blood glucose levels during a glucose tolerance test, wherein (c) is a case with administration of corosolic acid, (d) is a case with administration of banaba leaf extract and (e) is a case with administration of a placebo.
Fig. 3 is a graph showing changes in blood glucose levels with administration of corosolic acid and a placebo in the absence of a glucose load.
Fig. 4 is a graph showing changes in blood insulin levels with administration of corosolic acid and a placebo in the absence of a glucose load.

### Best Modes for Carrying Out the Invention

Corosolic acid, acyloxycorosolic acids, maslinic acid, acyloxymaslinic acids, ursolic acid and oleanolic acid, which are contained in the early insulin secretion stimulator of the invention, may be produced from banaba extract or banaba extract concentrate, or it may be extracted and purified from various types of plants such as loquat, mulberry or guava.

Corosolic acid, in particular, may be obtained by a variety of methods. Methods for its production and purification include extraction methods, as well as plant culturing, liquid culturing, microorganic application, enzyme utilization, semisynthesis, synthesis and gene manipulation.

Extract of banaba, as a typical plant containing abundant amounts of corosolic acid, may be obtained by extraction from banaba using hot water, an alcohol such as methanol, ethanol or propanol, or an aqueous solution of such alcohols. Banaba extract can also be obtained by immersion in cold water or in a solution of 100% ethanol. The principal components of extract obtained in this manner will be corosolic acid and banaba polyphenols (tannins in banaba leaves, flowers, stalks and the like). The extraction may be carried out by the following method.

Banaba leaves used as the raw material for banaba extract may be the fresh or dried leaves of banaba (*Lagerstroemia speciosa Linn.* or *Pers.)* produced in the Philippines, for example. Fresh leaves may be dried by natural drying, air drying or forced drying. The drying is carried out to a "toasted dry" state with a moisture content of no greater than 20 wt% and preferably no greater than 10 wt% in order to prevent growth of microorganisms and ensure storage stability.

The dried banaba leaves may be extracted directly, but they may instead be extracted after pulverization and chopping. There are no particular restrictions on the methods and conditions for hot water or alcohol extraction and concentration from the dried banaba leaves, but preferably methods and conditions are applied which will yield a constant proportion of corosolic acid in the concentrate. Specifically, when the banaba extract is processed into the banaba extract concentrate described hereunder, the corosolic acid content is preferably 0.1-15 mg per 100 mg of concentrate. The corosolic acid content is more preferably 0.2-12 mg and most preferably 0.5-10 mg per 100 mg of concentrate. Suitable extraction methods and conditions are described below.

Method 1: Ethanol or an aqueous ethanol solution (50-80 wt% ethanol content) is added to dried pulverized banaba leaves (raw material) at 5-20 times by weight and preferably 8-10 times by weight with respect to the raw material, and the mixture is heated to reflux or subjected to Soxhlet extraction from ordinary temperature to 90°C and preferably from about 50-85°C, for a period from 30 minutes to 2 hours. The extraction is repeated 2 or 3 times.

Method 2: Methanol or an aqueous methanol solution (50-90 wt% methanol content) is added at 3-20 times by weight to dried pulverized banaba leaves, and the mixture is heated to reflux or subjected to Soxhlet extraction in the same manner as Method 1. The extraction procedure is preferably carried out at a temperature from ordinary temperature to 65°C for a period from 30 minutes to 2 hours. The extraction procedure may be carried out once or repeated two or more times.

Method 3: Hot water is added at 3-20 times by weight to dried pulverized banaba leaves, and the mixture is heated to reflux or subjected to Soxhlet extraction at a temperature from 50-90°C and preferably 60-85°C, for a period from 30 minutes to 2 hours.

Methods 1 to 3 for extraction from banaba leaves may also be used in appropriate combinations. For example, Methods 1 and 2 may be carried out in combination. Preferred among these methods are Methods 1 and 2, with Method 1 being particularly preferred.

Banaba extract is usually processed into a banaba extract concentrate by concentration and drying for easier handling. The concentration and drying after extraction are preferably carried out in a relatively short time because storage of concentrates at high temperature for long periods can result in deterioration of the active components. It is therefore advantageous to perform the concentration and drying under reduced pressure. The extract obtained by the method described above is filtered and concentrated under reduced pressure at a temperature below 60°C, and the obtained solid is dried under reduced pressure at a temperature of 50-70°C (under a higher reduced pressure than for concentration). The solid obtained in this manner is pulverized to obtain a powdered concentrate. The banaba extract concentrate may be processed into the form of a tablet or a granule instead of a powder. A banaba extract concentrate obtained by such a method comprises corosolic acid, banaba polyphenols and other active components.

A publicly known extraction method (for example, a method involving liquid chromatography, such as HPLC) may be used to remove the components other than corosolic acid (other components) from the banaba extract or banaba extract concentrate obtained in the manner described above, in order to obtain corosolic acid (corosolic acid content of 99% or greater). Maslinic acid, ursolic acid and oleanolic acid can be obtained from the other components obtained from banaba extract or banaba extract concentrate.

Corosolic acid (or maslinic acid) may be used directly, but it may also be acylated (for example, acetylated), or subsequently deacylated (for example, deacetylated). Most preferably, corosolic acid (or maslinic acid) is acylated (for example, acetylated) and then deacylated. By removing the acyl groups from acylated corosolic acid it is possible to obtain corosolic acid of very high purity (approximately 100%).

The method of acetylating corosolic acid may be carried out, for example, by dissolving the obtained corosolic acid in anhydrous pyridine, adding acetic anhydride and allowing the mixture to stand at room temperature for about 12 hours, and then adding ice water to the reaction mixture, performing extraction several times (about 3 times) with chloroform, dewatering the chloroform layer with anhydrous sodium sulfate, removing the sodium sulfate by filtration, distilling off the chloroform under reduced pressure, and recrystallizing from hexane.

As an example of a method of deacylating corosolic acid there may be mentioned a method of hydrolysis with an alkali such as potassium hydroxide or sodium hydroxide.

The corosolic acid, acyloxycorosolic acids, maslinic acid, acyloxymaslinic acids, ursolic acid, oleanolic acid, acyloxyursolic acids or acyloxyoleanolic acids obtained in the manner described above may be used for production of pharmaceuticals, health supplements, food materials, smoking materials and animal feeds, by application of publicly known methods.

The health supplement may be produced by subjecting banaba leaves or a corosolic acid-containing plant to hot water extraction or alcohol extraction to draw out the active components, and incorporating the dried powder or liquid into a health supplement. The health supplement may be used in tablet, capsule, application, suppository, nasal drop, injection, granule or powder form.

The food material may be produced by subjecting banaba leaves or a corosolic acid-containing plant to hot water extraction or alcohol extraction to draw out the active components, and incorporating the dried powder or liquid into a food material. Incidentally, since food materials are heavily evaluated based on color, flavor and aroma, they are preferably used in the form of extracts with the tannins and chlorophyll removed from the active components, or powders obtained by dilution of the extracts with starch or dextrin. Such food materials may be used as portions of raw materials in the same manner as ordinary wheat flour, starch, sugar, salt, soy sauce or cooking oils and fats, or as cooking materials, flavorings or the like.

The smoking materials refers to tobacco products, for example, and include smoking materials used in any form such as cigarettes, cigars, pipes, smoking tubes and the like. Specifically, banaba leaves may be finely chopped, and either mixed with tobacco leaves of the same size or used alone as a smoking material for use in the form of a cigarette or cigar. Alternatively, banaba extract may be dissolved in ethanol or another alcohol, and tobacco leaves immersed therein and dried for convenient use as cigar or cigarette tobacco. These smoking materials are burned with a flame, and therefore also exhibit the effects of the volatile components generated at high temperature in proximity to the flame, or the volatile components generated by burning. That is, inhalation of these volatile components from the air space can result in a significant effect even if the corosolic acid is only included in a trace amount. Thus, the habit of smoking may be utilized as a method for oral absorption of corosolic acid.

The animal feed according to the invention also includes fish feed, and it may be given to livestock or pets for treatment or prevention of animal diabetes, hypertension, hyperlipidemia and obesity, for maintenance of animal health. Specifically, it may be produced by subjecting banaba leaves or a plant containing corosolic acid to hot water extraction or to alcohol extraction to draw out the active components, drying the extract to obtain a powder or otherwise removing the liquid for inclusion into a pet food, or into feed for poultry or livestock such as chickens, pigs, cows or goats, or cultivated fish. The animal feed may be used for food in the same manner as ordinary feed or pet food.

### Examples

The present invention will now be explained in greater detail through examples and comparative examples, with the understanding that these examples are in no way limitative on the invention.

(Diacetylcorosolic acid Production Example 1)
Dry banaba leaves were pulverized and extracted with hot ethanol, and then concentrated under reduced pressure to obtain an ethanol extract. The ethanol extract was suspended in water and then extracted with hexane to obtain a hexane extract. The aqueous layer was subjected to DIAION HP-20 column chromatography and eluted stepwise with water, 50% methanol and methanol, the fractions of which were subjected to solvent distillation under reduced pressure.

The methanol-extracted fraction was dissolved in anhydrous pyridine, and then acetic anhydride was added and the mixture was stirred at room temperature for 24 hours. Ice water was then added to the reaction mixture, and extraction was performed 3 times with chloroform. The chloroform layer was dewatered with anhydrous magnesium sulfate, and then the magnesium sulfate was removed by filtration and the chloroform was distilled off under reduced pressure.

The obtained residue was separated by silica gel column chromatography (dichloromethane:methanol = 150: 1) and purification was performed by high performance liquid chromatography (hexane:2-propanol = 99:1) using a normal phase column to obtain diacetylcorosolic acid.

(Diacetylcorosolic acid Production Example 2)
Dry banaba leaves were pulverized and extracted with hot ethanol, and then concentrated under reduced pressure to obtain an ethanol extract. The ethanol extract was suspended in water and then extracted with hexane to obtain a hexane extract. The aqueous layer was subjected to DIAION HP-20 column chromatography and eluted stepwise with water, 50% methanol and methanol, the fractions of which were subjected to solvent distillation under reduced pressure.

The methanol-extracted fraction was dissolved in anhydrous pyridine, and then acetic anhydride was added and the mixture was allowed to stand at room temperature for 12 hours. Ice water was then added to the reaction mixture, and extraction was performed 3 times with chloroform. The chloroform layer was dewatered with anhydrous sodium sulfate, and then the sodium sulfate was removed by filtration and the chloroform was distilled off under reduced pressure. The obtained residue was recrystallized from hexane to obtain diacetylcorosolic acid (colorless needle-like crystals).

(Confirmation of diacetylcorosolic acid)
The diacetylcorosolic acid obtained by each of the diacetylcorosolic acid Production Examples 1 and 2 was confirmed using thin-layer chromatography (TLC). Silica gel 60 F254 (Merck) was used as the silica gel and chloroform:methanol = 20:1 was used as the developing solvent. The Rf values for corosolic acid and diacetylcorosolic acid were 0.21 (corosolic acid) and 0.59 (diacetylcorosolic acid), and the results of TLC confirmed that none of the corosolic acid raw material was left.

(Diacetylmaslinic acid Production Example 1)
Dry banaba leaves were pulverized and extracted with hot ethanol, and then concentrated under reduced pressure to obtain an ethanol extract. The ethanol extract was suspended in water and then extracted with hexane to obtain a hexane extract. The aqueous layer was subjected to DIAION HP-20 column chromatography and eluted stepwise with water, 50% methanol and methanol, the fractions of which were subjected to solvent distillation under reduced pressure.

The methanol-extracted fraction was dissolved in anhydrous pyridine, and then acetic anhydride was added and the mixture was stirred at room temperature for 24 hours. Ice water was then added to the reaction mixture, and extraction was performed 3 times with chloroform. The chloroform layer was dewatered with anhydrous magnesium sulfate, and then the magnesium sulfate was removed by filtration and the chloroform was distilled off under reduced pressure.

The obtained residue was separated by silica gel column chromatography (dichloromethane:methanol = 150: 1) and purification was performed by high performance liquid chromatography (hexane:2-propanol = 99:1) using a normal phase column to obtain diacetylmaslinic acid.

(Diacetylmaslinic acid Production Example 2)
Dry banaba leaves were pulverized and extracted with hot ethanol, and then concentrated under reduced pressure to obtain an ethanol extract. The ethanol extract was suspended in water and then extracted with hexane to obtain a hexane extract. The aqueous layer was subjected to DIAION HP-20 column chromatography and eluted stepwise with water, 50% methanol and methanol, the fractions of which were subjected to solvent distillation under reduced pressure.

The methanol-extracted fraction was dissolved in anhydrous pyridine, and then acetic anhydride was added and the mixture was allowed to stand at room temperature for 12 hours. Ice water was then added to the reaction mixture, and extraction was performed 3 times with chloroform. The chloroform layer was dewatered with anhydrous sodium sulfate, and then the sodium sulfate was removed by filtration and the chloroform was distilled off under reduced pressure. The obtained residue was recrystallized from hexane to obtain diacetylmaslinic acid (colorless needle-like crystals).

(Confirmation of diacetylmaslinic acid)
The diacetylmaslinic acid obtained by each of the diacetylmaslinic acid Production Examples 1 and 2 was confirmed using thin-layer chromatography (TLC). Silica gel 60 F254 (Merck) was used as the silica gel and chloroform:methanol = 20:1 was used as the developing solvent. The Rf values for maslinic acid and diacetylmaslinic acid were 0.21 (maslinic acid) and 0.59 (diacetylmaslinic acid), and the results of TLC confirmed that none of the maslinic acid raw material was left.

(Deacetylation of diacetylcorosolic acid (or diacetylmaslinic acid))
The diacetylcorosolic acid (or diacetylmaslinic acid) was added to a 1 N potassium hydroxide methanol solution and the mixture was allowed to stand at room temperature for 2 hours for deacetylation. This was followed by neutralization with an ion-exchange membrane to obtain high purity corosolic acid (or maslinic acid).

(Confirmation of high purity corosolic acid (or maslinic acid))
The purity of the obtained high purity corosolic acid (or maslinic acid) was confirmed by high performance liquid chromatography. The conditions employed were MEH:0.05% TFA = 85:15 as the mobile phase and YMCPACKODS (4.6 mm I.D x 250 mm) as the column. The UV (210 nm) of the obtained component was measured to confirm a corosolic acid (or maslinic acid) purity of about 100%.

(Test Example 1: Confirmation of early insulin secretion stimulating effect)
The following test was conducted to confirm that the obtained corosolic acid had an early insulin secretion stimulating effect. A glucose tolerance test was performed after administration of corosolic acid or a placebo, in a double-blind crossover manner, and the blood insulin level (IRI: immunoreactive insulin) was assayed.

First, 31 borderline diabetic patients as test subjects were orally administered corosolic acid (10 mg, ≥99% purity) or a placebo, and blood was sampled immediately thereafter with the blood insulin level at that time designated as the value at 0 minutes. Immediately after blood sampling, 75 g of glucose was orally administered to each test subject to start a glucose tolerance test, and blood was sampled after periods of 30 minutes, 60 minutes, 90 minutes, 120 minutes and 180 minutes for measurement of the blood insulin level.

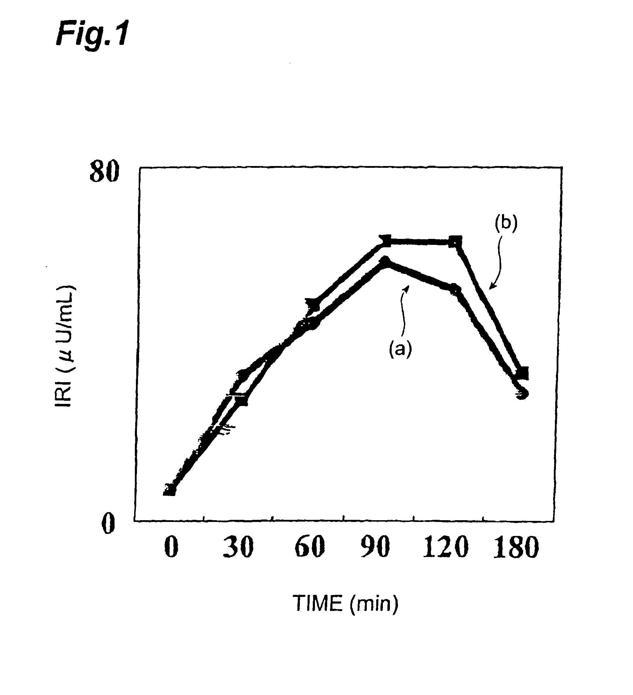

Fig. 1 is a graph showing the blood insulin levels during the glucose tolerance test. The horizontal axis represents time (min) after start of the glucose tolerance test, and the vertical axis represents IRI (µU/mL). In the graph, (a) is a case with administration of corosolic acid and (b) is a case with administration of the placebo.

As seen by the results in Fig. 1, administration of corosolic acid produced a significant increase in blood insulin level (p<0.05) at 30 minutes after start of the glucose tolerance test, compared to administration of the placebo. However, a significant drop in blood insulin level (p<0.05) was seen at 120 minutes after start of the glucose tolerance test.

This demonstrated that corosolic acid stimulates early insulin secretion. It was also shown that no excess insulin was secreted in the absence of blood glucose increase.

(Test Example 2: Confirmation of hypoglycemic effect)
The following test was conducted to confirm that corosolic acid has a hypoglycemic effect. A glucose tolerance test was performed after administration of corosolic acid, banaba extract or a placebo, in a double-blind crossover manner, and the blood glucose level (plasma glucose concentration) was assayed.

First, 35 test subjects were orally administered corosolic acid (10 mg, ≥99% purity), banaba extract (trade name: COROSOLIA M, product of Use Techno Corporation; administered at 10 mg of corosolic acid) or a placebo, and blood was sampled immediately thereafter with the blood glucose level at that time designated as the value at 0 minutes. Immediately after blood sampling, 75 g of glucose was orally administered to each test subject to start a glucose tolerance test, and blood was sampled after periods of 30 minutes, 60 minutes, 90 minutes, 120 minutes and 180 minutes for measurement of the blood glucose level.

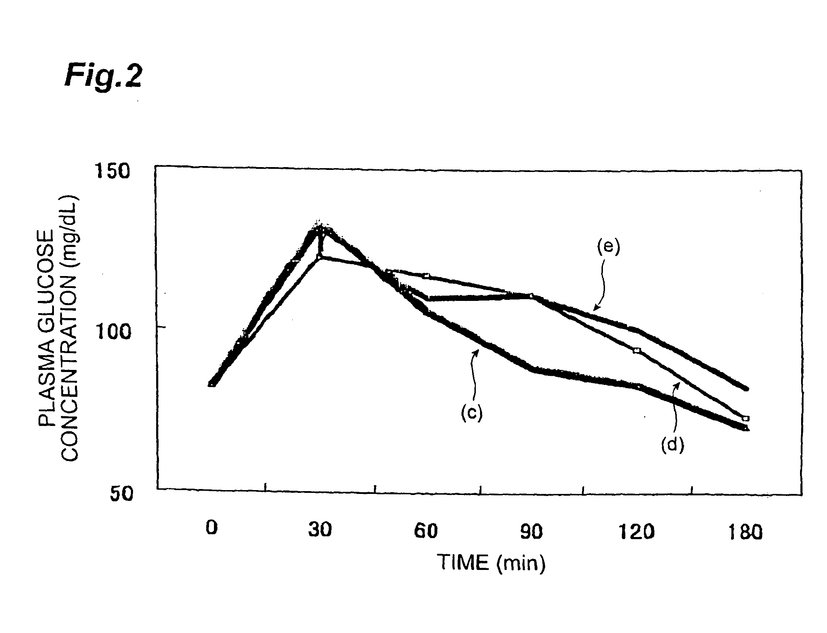

Fig. 2 is a graph showing blood glucose levels during the glucose tolerance test. The horizontal axis represents time (min) after start of the glucose tolerance test, and the vertical axis represents plasma glucose concentration (mg/dL). In the graph, (c) is a case with administration of corosolic acid, (d) is a case with administration of banaba leaf extract and (e) is a case with administration of the placebo.

As seen by the results in Fig. 2, administration of corosolic acid produced a significant decrease in blood glucose level (p<0.05) at 90 and 120 minutes after start of the glucose tolerance test, compared to administration of the placebo. This demonstrated that corosolic acid exhibits a hypoglycemic effect.

(Test Example 3: Confirmation of glucose-dependent early insulin secretion stimulating effect)
Three borderline diabetic patients (55-yr-old male, 54-yr-old male and 45-yr-old male) were instructed to fast from 8:00 pm (to ensure absence of glucose load), and were administered a placebo once or the test agent (corosolic acid) once at 9:00 am on the following day, after which the changes in blood glucose levels and blood insulin levels were measured. The test subjects were given absolutely no information regarding placebo or test agent (crossover, double-blind). The corosolic acid was administered in a single 10 mg dose, and each test was conducted twice.

The test results are shown in Table 1.

**[Table 1]**

| Test subject | H | W | A | | After 0 min | After 30 min | After 60 min | After 90 min | After 120 min | After 180 min | Administered agent |
|---|---|---|---|---|---|---|---|---|---|---|---|
| A(M) | 168 | 76 | 55 | | G 111 | 111 | 110 | 112 | 108 | 106 | PCB |
| | | | | 1 | 10.5 | 7.9 | 7.8 | 11.2 | 9.7 | 8.2 | PCB |
| | | | | G | 110 | 104 | 107 | 104 | 104 | 102 | CRA |
| | | | | I | 9.0 | 6.9 | 6.6 | 5.9 | 6.6 | 5.1 | CRA |
| B(M) | 167 | 60 | 54 | G | 150 | 147 | 149 | 157 | 165 | 171 | PCB |
| | | | | 1 | 3.3 | 4.5 | 2.4 | 2.6 | 2.4 | 2.1 | PCB |
| | | | | G | 144 | 143 | 147 | 149 | 150 | 146 | CRA |
| | | | | 1 | 2.5 | 2.6 | 3.0 | 2.0 | 3.4 | 2.4 | CRA |
| C(M) | 166 | 65 | 45 | G | 100 | 105 | 101 | 101 | 100 | 94 | PCB |
| | | | | 1 | 8.7 | 5.8 | 9.0 | 6.3 | 6.6 | 5.3 | PCB |
| | | | | G | 90 | 90 | 88 | 86 | 86 | 81 | CRA |
| | | | | 1 | 4.5 | 4.1 | 3.9 | 4.9 | 4.0 | 4.0 | CRA |

| | | | | | | | | | | | |
|---|---|---|---|---|---|---|---|---|---|---|---|
| (Notes) M: male, H: body height, W: body weight, A: age, G: blood glucose level. I: blood insulin level, PCB: placebo, CRA: corosolic acid | | | | | | | | | | | |

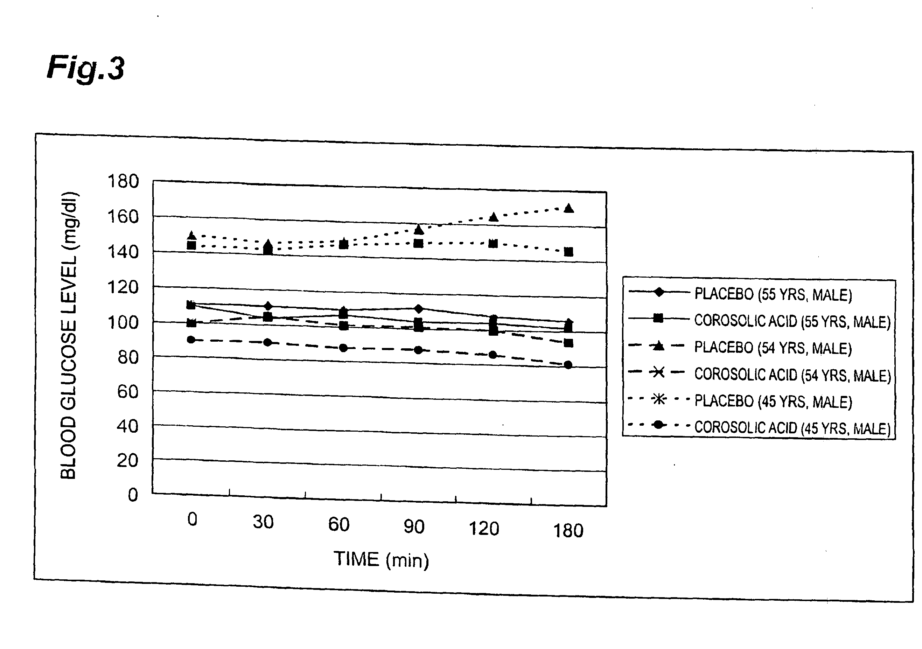

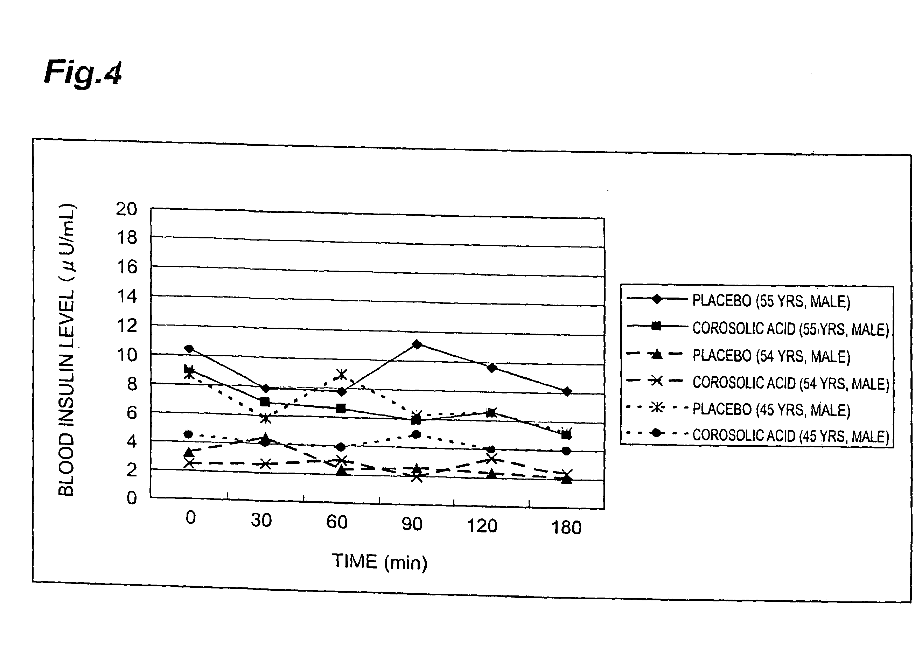

Fig. 3 is a graph showing changes in blood glucose levels with administration of corosolic acid and a placebo in the absence of a glucose load, and Fig. 4 is a graph showing changes in blood insulin levels with administration of corosolic acid and a placebo in the absence of a glucose load. In Figs. 3 and 4, no significant difference is seen in blood glucose levels and blood insulin levels with administration of the corosolic acid or placebo, thereby demonstrating that the early insulin secretion stimulator of the invention is a glucose-dependent early insulin secretion stimulator.

(Test Example 4: Preparation of health supplements)
[Product 1: Tablets and drink]
Corosolic acid was added to a tablet or drink at a corosolic acid content of 0.18 wt% with respect to the total weight of the tablet or drink.

This concentration is suitable for trace elements or other nutrients, and is particularly effective for persons concerned about blood glucose. Dilution may be performed in accordance with the use, purpose, form and amount.

[Product 2: Yogurt]
A corosolic acid-containing yogurt was prepared having a corosolic acid content of 1 mg per 100 g yogurt pack. For non-sugar yogurt, corosolic acid was added for a corosolic acid content of 1 mg to 3.9 g of added sugar weight, for example.

(Test Example 5: Preparation of food materials)
[Product 3: Bread]
Bread was prepared with the recipe shown in Table 2, for a corosolic acid intake of 1 mg per serving.

**[Table 2]**

| | |
|---|---|
| Recipe | Amount |
| Hard flour | 300 g |
| Dry yeast | 6 g |
| Salt | 5 g |
| Corosolic acid | 0.1 g |
| Sugar | 10 g |
| Tepid water | 200 cc |
| Skim milk | 6 g |
| Butter | 20 g |

<Preparation Method>
The hard flour, dry yeast, salt, corosolic acid, sugar, skim milk and tepid water were combined, and the mixture was kneaded while adding in the butter. When the mixture began to harden, kneading was continued for 15 minutes to prepare dough.

The dough was fermented at 40°C for 60 minutes, and after rising sufficiently, the dough was separated into two portions and the internal gas was removed. The gas-removed dough was rolled into a ball and placed in a bread mold and allowed to stand for 10 minutes. The dough was fermented once more at 40°C for 30 minutes, and upon reaching a volume of 2-3 times it was baked at 170°C for 25 minutes to obtain bread.

[Product 4: Cream puff]
Twelve cream puffs were prepared with the recipe shown in Table 3 and Table 4, for a corosolic acid intake of 1 mg per cream puff.

**[Table 3]**

| | |
|---|---|
| Recipe (puff dough) | Amount |
| Water | 60 cc |
| Milk | 60 cc |
| Salt-free butter | 50 g |
| Salt | small amount |
| Wheat flour/soft flour | 60 g |
| Chicken egg | 2 eggs |

**[Table 4]**

| | |
|---|---|
| Recipe (custard cream) | Amount |
| Egg yolk | 3 yolks |
| Corosolic acid | 0.12 g |
| Granulated sugar | 50 g |
| Wheat flour/soft flour | 12 g |
| Corn starch | 12 g |
| Milk | 200 cc |
| Salt-free butter | 10 g |
| Vanilla essence | small amount |
| Curacao (cointreau) | 20 cc |
| Fresh cream cup | 50 cc |
| Granulated sugar | 5 g |

<Preparation Method>
First, the milk, water, butter and salt for the dough were placed in a pot and heated. Heating was terminated when the butter melted, and after adding the soft flour, the mixture was heated again for 2-3 minutes. The beaten eggs were added and mixed therewith, and the dough was squeezed out into disks on an oven paper-covered pan. After adjusting the shapes, the mixture was baked in an oven at 190°C for 15 minutes, and then the temperature was lowered from 180°C to 160°C for 15 minutes of baking. This was followed immediately by drying for 5 minutes to obtain puff dough.

For the custard cream, the milk, corosolic acid and sugar were placed in a pot and heated. Heating was suspended just before boiling, and then the vanilla essence and cointreau were added. The powders and beaten egg yolks were mixed therewith, and when the mixture became smooth the butter was mixed in and the mixture was placed in a refrigerator for cooling. The whipped fresh cream was then mixed into the cooled mixture to obtain custard cream.

The custard cream was filled into the baked puff dough, and the powdered sugar was sprinkled over as finishing to prepare cream puffs.

[Product 5: Other foods]
Other foods including chocolate, pudding, cheesecake, salad dressing, seasoning, soy sauce and soybean paste may be prepared with addition of corosolic acid dextrin instead of sugar. The intake of corosolic acid is preferably 1 mg per serving.

### Industrial Applicability

There are provided an early insulin secretion stimulator which stimulates early secretion of insulin to lower postprandial blood glucose levels while preventing excess insulin secretion; a process for its production; and a pharmaceutical, a health supplement, a smoking material, a food material and an animal feed which employ the early insulin secretion stimulator.

## Claims

1. An early insulin secretion stimulator comprising one or more medicinal components selected from the group consisting of corosolic acid, acyloxycorosolic acids, maslinic acid and acyloxymaslinic acids at a content of at least 99 wt% of the total weight.

2. The early insulin secretion stimulator according to claim 1, wherein the one or more medicinal components selected from the group consisting of corosolic acid, acyloxycorosolic acids, maslinic acid and acyloxymaslinic acids is corosolic acid.

3. The early insulin secretion stimulator according to claim 1, wherein the one or more medicinal components selected from the group consisting of corosolic acid, acyloxycorosolic acids, maslinic acid and acyloxymaslinic acids is maslinic acid.

4. The early insulin secretion stimulator according to claim 1, which contains said medicinal components at 100 wt% of the total weight.

5. The early insulin secretion stimulator according to claim 1, which contains corosolic acid at 99 wt% or greater of the total weight, and maslinic acid at less than 1 wt% of the total weight.

6. The early insulin secretion stimulator according to claim 1, wherein said acyloxycorosolic acid is acetylcorosolic acid.

7. The early insulin secretion stimulator according to claim 1, wherein said acyloxymaslinic acid is acetylmaslinic acid.

8. An early insulin secretion stimulator consisting of a glucoside of the early insulin secretion stimulator according to claim 1.

9. An early insulin secretion stimulator consisting of an ester of the early insulin secretion stimulator according to claim 1.

10. An early insulin secretion stimulator consisting of 100 parts by weight of the early insulin secretion stimulator according to claim 1, and 1-99 parts by weight of ursolic acid and oleanolic acid.

11. The early insulin secretion stimulator according to claim 1, which is glucose-dependent.

12. A pharmaceutical comprising the early insulin secretion stimulator according to claim 1.

13. A smoking material comprising the early insulin secretion stimulator according to claim 1.

14. A health supplement comprising the early insulin secretion stimulator according to claim 1.

15. A food material obtained by adding the early insulin secretion stimulator according to claim 1 to a material selected from the group consisting of bread, noodles, confectioneries, beverages, sugar, alcoholic beverages, fats and oils, wheat flour, starches, and the like.

16. An animal feed comprising the early insulin secretion stimulator according to claim 1.

17. A process for production of high purity corosolic acid, which includes a step of deacylating an acyloxycorosolic acid represented by the following general formula (1). 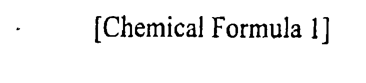 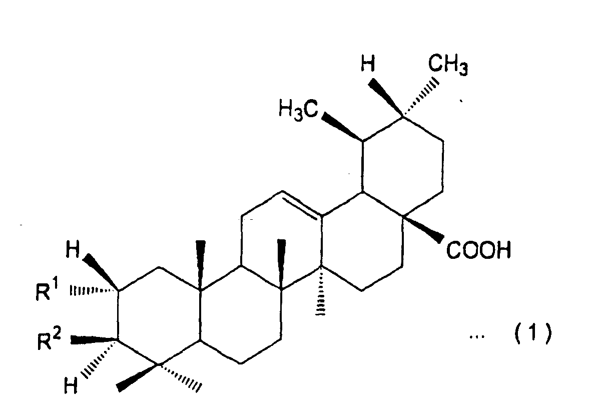 [In formula (1), R¹ and R² each independently represent an acyloxy group or hydroxyl group, with the proviso that R¹ and R² are not both hydroxyl groups.]

18. A process for production of high purity maslinic acid, which includes a step of deacylating an acyloxymaslinic acid represented by the following general formula (2). 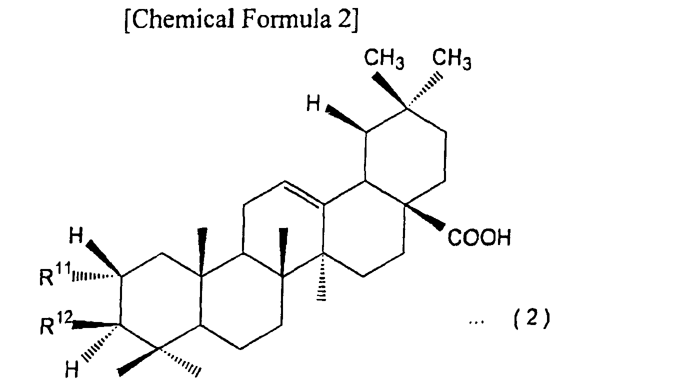 [In formula (2), R¹¹ and R¹² each independently represent an acyloxy group or hydroxyl group, with the proviso that R¹¹ and R¹² are not both hydroxyl groups.]
